# EUROPÄISCHE PATENTSCHRIFT

(11) **EP 0 689 417 B1**
(45) Veröffentlichungstag und Bekanntmachung des Hinweises auf die Patenterteilung: **17.09.1997**
(21) Anmeldenummer: 94911905.1
(22) Anmeldetag: 16.03.1994
(51) Int. Cl.: A61K 7/09

(54) **VERFAHREN UND MITTEL ZUR DAUERHAFTEN VERFORMUNG VON KERATINFASERN**
PROCESS AND AGENT FOR THE PERMANENT DEFORMATION OF KERATIN FIBRES
PROCEDE ET AGENTS DE DEFORMATION PERMANENTE DE FIBRES DE KERATINE

(30) Priorität: 25.03.1993 DE 4309509
(43) Veröffentlichungstag der Anmeldung: 03.01.1996
(73) Patentinhaber: Henkel Kommanditgesellschaft auf Aktien, 40191 Düsseldorf (DE)
(72) Erfinder: BUSCH, Peter, D-40699 Erkrath (DE); FISCHER, Detlef, D-41372 Niederkrüchten (DE); STRIEPLING, Gert-Lothar, D-40591 Düsseldorf (DE)
(86) Internationale Anmeldenummer: EP9400837
(87) Internationale Veröffentlichungsnummer: WO9421218

(56) Entgegenhaltungen:
- EP-A- 0 394 920
- DE-A- 1 492 094
- DE-A- 3 740 926

## Beschreibung

Die Erfindung betrifft ein Verfahren zur dauerhaften Verformung von Keratinfasern, insbesondere von menschlichen Haaren, durch reduktive Spaltung und erneute oxidative Knüpfung von Disulfidbindungen des Keratins sowie für dieses Verfahren geeignete Mittel.

Die dauerhafte Verformung von Keratinfasern wird üblicherweise so durchgeführt, daß man die Faser mechanisch verformt und die Verformung durch geeignete Hilfmittel festlegt. Vor und/oder nach dieser Verformung behandelt man die Faser mit der wäßrigen Zubereitung einer keratinreduzierenden Substanz und spült nach einer Einwirkungszeit mit Wasser oder einer wäßrigen Lösung. In einem zweiten Schritt behandelt man dann die Faser mit der wäßrigen Zubereitung eines Oxidationsmittels. Nach einer Einwirkungszeit wird auch dieses ausgespült und die Faser von den mechanischen Verformungshilfsmitteln (Wickler, Papilloten) befreit.

Die wäßrige Zubereitung des Keratinreduktionsmittels ist üblicherweise alkalisch eingestellt, damit die Faser quillt und auf diese Weise ein tiefes Eindringen der keratinreduzierenden Substanz in die Faser ermöglicht wird. Die keratinreduzierende Substanz spaltet einen Teil der Disulfid-Bindungen des Keratins zu -SH-Gruppen, so daß es zu einer Lockerung der Peptidvernetzung und infolge der Spannung der Faser durch die mechanische Verformung zu einer Neuorientierung des Keratingefüges kommt. Unter dem Einfluß des Oxidationsmittels werden erneut Disulfid-Bindungen geknüpft, und auf diese Weise wird das Keratingefüge in der vorgegebenen Verformung neu fixiert.

Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten Haaren angewendet werden.

Eine negative Begleiterscheinung der so durchgeführten Dauerwellung des Haares ist aber regelmäßig ein Verspröden und Stumpfwerden der Haare.

Weiterhin werden in vielen Fällen auch andere Eigenschaften wie Naß- und Trockenkämmbarkeit, Griff, Geschmeidigkeit, Weichheit, Glanz und Reißfestigkeit in ungewünschter Weise beeinflußt.

Es hat daher in der Vergangenheit nicht an Versuchen gefehlt, hier für Abhilfe zu sorgen.

Eine entsprechende Modifizierung der reduzierenden Lösung führt zu in der Regel nicht befriedigenden Welleistungen. Die Zugabe bekannter Zusätze wie Strukturanten, Polymere, Filmbildner und vernetzende Harze oder die neutrale bis schwach saure Einstellung der Zubereitung kann die Schädigung des Haares zwar verringern; jedoch bleibt das Haar in seiner Struktur mehr oder weniger geschwächt.

Es bestand daher weiterhin die Aufgabe, ein Verfahren der dauerhaften Verformung von Keratinfasern zu finden, bei welchem die genannten unerwünschten Nebenwirkungen weiter reduziert oder ganz ausgeschlossen werden.

Es wurde nun überraschenderweise gefunden, daß eine wesentliche Verbesserung der Eigenschaften, insbesondere der Reißfestigkeit, verformter Keratinfasern dadurch erreicht wird, daß den während des Verformungsverfahrens eingesetzten Mitteln spezielle Verbindungen zugesetzt werden.

Gegenstand der Erfindung ist daher ein Verfahren zur dauerhaften Verformung von Keratinfasern, bei welchem man die Faser vor und/oder nach einer mechanischen Verformung mit einer wäßrigen Zubereitung einer keratinreduzierenden Substanz behandelt, nach einer Einwirkungszeit mit einer ersten Spülung spült, dann mit einer wäßrigen Zubereitung eines Oxidationsmittels fixiert und ebenfalls nach einer Einwirkungszeit spült, dadurch gekennzeichnet, daß in mindestens einer der beiden wäßrigen Zubereitungen oder in der ersten Spülung ein Tocopherol oder ein Ester eines Tocopherols mit einer organischen oder anorganischen Säure enthalten ist.

Im weiteren werden folgende Bezeichnungen verwendet:
- "Wellmittel" für die wäßrige Zubereitung der keratinreduzierenden Substanz,
- "Zwischenspülung" für die erste Spülung und
- "Fixiermittel" für die wäßrige Zubereitung des Oxidationsmittels.

Weiterhin sollen die Begriffe "Tocopherol" bzw. "Tocopherole" im folgenden sowohl für die verschiedenen Tocopherole selbst als auch für deren Ester mit organischen und anorganischen Säuren stehen.

Die Verwendung von Vitamin E (Tocopherol und Tocopherol-Derivaten) in der Kosmetik, insbesondere zur Hautbehandlung, ist bekannt. Weiterhin war bekannt, z.B. aus Ärztliche Kosmetologie 19, 193-207 (1989), daß Vitamin E-haltige Spülungen präventiv gegen Haarschädigungen durch starke Sonnenbestrahlung (Ausbleichen, verringerte Reißfestigkeit) wirken. Dem Stand der Technik sind aber keine Informationen über mögliche positive Wirkungen von Tocopherol-Zusätzen in Verfahren zur Verformung von Keratinfasern zu entnehmen.

Alle bekannten natürlichen und synthetischen Tocopherole können in dem erfindungsgemäßen Verfahren verwendet werden, z.B. α-, β-, γ-, δ-, ε-Tocopherol und entsprechende Racemate. Die Verwendung von α-Tocopherol, insbesondere D-α-Tocopherol, ist bevorzugt.

Erfindungsgemäß bevorzugte Tocopherol-Ester mit organischen Säuren sind Ester der genannten Tocopherole mit Mono- und Dicarbonsäuren wie Tocopherylacetat, Tocopherylpalmitat, Tocopheryllinoleat, Tocopherylsuccinat, Tocopherylpoly(oxyethylen)succinat und Tocopherylnicotinat. α-Tocopherylacetat, α-Tocopherylpalmitat und α-Tocopherylsuccinat sind besonders bevorzugte Ester.

Ein bevorzugter Ester mit einer anorganischen Säure ist das Tocopherylphosphat, insbesondere das α-Tocopherylphosphat.

Die Tocopherole sind in den für das erfindungsgemfäße Verfahren verwendeten Zubereitungen bevorzugt in Mengen von 0,1 bis 10, insbesondere von 1 bis 3 Gew.-%, bezogen auf die jeweilige Zubereitung, enthalten.

Im Rahmen der erfindungsgemäßen Lehre können die Tocopherole sowohl im Wellmittel als auch in der Zwischenspülung oder im Fixiermittel enthalten sein. Es hat sich als besonders vorteilhaft erwiesen, wenn die Tocopherole Bestandteil des Wellmittels sind und gewünschtenfalls zusätzlich in der Zwischenspülung oder dem Fixiermittel enthalten sind.

Weiterer Gegenstand der Anmeldung sind die zur Durchführung des erfindungsgemäßen Verfahrens verwendbaren Mittel.

Diese Mittel dienen im Rahmen eines Verfahrens zur dauerhaften Verformung von Keratinfasern entweder zur Durchführung der reduzierenden Stufe, zur Durchführung der oxidierenden Stufe oder zum Spülen nach Durchführung der reduzierenden Stufe und enthalten neben für diese Mittel üblichen Bestandteilen zusätzlich ein Tocopherol oder einen Ester eines Tocopherols mit einer organischen oder anorganischen Säure.

Die erfindungsgemäßen Mittel können alle bekannten natürlichen und synthetischen Tocopherole enthalten, z.B. α-, β-, γ-, δ-, ε-Tocopherol und entsprechende Racemate. Die Verwendung von α-Tocopherol, insbesondere D-α-Tocopherol, ist bevorzugt.

Für die erfindungsgemäßen Mittel bevorzugte Tocopherol-Ester mit organischen Säuren sind Ester der genannten Tocopherole mit Mono- und Dicarbonsäuren wie Tocopherylacetat, Tocopherylpalmitat, Tocopheryllinoleat, Tocopherylsuccinat, Tocopherylpoly(oxyethylen)succinat und Tocopherylnicotinat. α-Tocopherylacetat, α-Tocopherylpalmitat und α-Tocopherylsuccinat sind besonders bevorzugte Ester.

Ein bevorzugter Ester mit einer anorganischen Säure ist das Tocopherylphosphat, insbesondere das α-Tocopherylphosphat.

Die Tocopherole sind in den erfindungsgemäßen Mitteln bevorzugt in Mengen von 0,1 bis 10, insbesondere von 1 bis 3 Gew.-%, bezogen auf das jeweilige Mittel, enthalten.

Bevorzugt werden die erfindungsgemäßen Mittel zum Dauerwellen bzw. Glätten von menschlichen Haaren verwendet. Sie enthalten dann weiterhin die für Mittel für Dauerwellverfahren bekannten weiteren Bestandteile.

Die erfindungsgemäßen Wellmittel enthalten zwingend die als keratinreduzierende Substanzen bekannten Mercaptane. Solche Verbindungen sind beispielsweise Thioglykolsäure, Thiomilchsäure, 3-Mercaptopropionsäure sowie deren Salze und Ester, Cysteamin, Cystein, Bunte Salze und Alkalisalze der schwefligen Säure. Bevorzugt geeignet sind die Alkali- oder Ammoniumsalze der Thioglykolsäure und/oder der Thiomilchsäure sowie die freien Säuren. Diese werden in den Wellmitteln bevorzugt in Konzentrationen von 0,5 bis 1,0 Mol/kg bei einem pH-Wert von 6,5 bis 10 eingesetzt. Die Einwirkungszeit der Wellmittel auf dem Haar beträgt in der Regel 20 bis 40 Minuten, wobei die Dicke des zu behandelnden Haares, der gewünschte Verformungsgrad, die Größe der verwendeten mechanischen Verformungshilfe (Haarwickler) und die Art des Keratinreduktionsmittels weitere Einflußgrößen sind.

Die erfindungsgemäßen Wellmittel können als gebrauchsfertige Mischungen formuliert werden, die vom Friseur oder Endverbraucher direkt angewendet werden können. Es hat sich in vielen Fällen, insbesondere bei Verwendung starker Reduktionsmittel, aber als vorteilhaft erwiesen, wenn die Mittel als sogenannte 2-Komponenten-Mischungen formuliert werden, die erst vom Anwender zum gebrauchsfertigen Wellmittel vermischt werden. In diesem Fall enthält eine Formulierung das Reduktionsmittel in einem geeigneten Träger, z.B. Wasser oder einer Emulsion. Die andere Formulierung enthält im gleichen oder einem ähnlichen Trägersystem die weiteren Komponenten einschließlich der Tocopherole.

Weiterhin hat es sich als vorteilhaft erwiesen, wenn die Tocopherole in den erfindungsgemäßen Mitteln in Kombination mit Protein-Derivaten eingesetzt werden.

Geeignete Protein-Derivate sind Proteinhydrolysate, Kondensationsprodukte dieser Proteinhydrolysate, insbesondere mit Säuren, sowie quaternisierte Proteinhydrolysate.

Proteinhydrolysate sind Produktgemische, die durch sauer, basisch oder enzymatisch katalysierten Abbau von Proteinen (Eiweißen) erhalten werden.

Erfindungsgemäß verwendbar sind beispielsweise folgende Hydrolysate:
- Kollagen-Hydrolysate (CTFA-Bezeichnungen: Hydrolyzed Animal Protein, Hydrolyzed Animal Collagen und Soluble Animal Collagen).
   Hydrolysate auf Basis Rinder-Kollagen können hierbei bevorzugt sein. Die Hydrolysate können auch in Form von Salzen, beispielsweise als Natrium- oder Ammoniumsalz vorliegen. Bekannte Handelsprodukte sind beispielsweise Dehylan^{R}ET 40 (Henkel), Promois^{R}Silk 1000 Q (Interorgana), Collapuron^{R}N (Henkel), Nutrilan^{R}V 37908 und Nutrilan^{R}I (Grünau), Gelita-Sol^{R}C und CS (Deutsche Gelatine Fabriken Stoess & Co) sowie Lexein^{R} LP 170 und Lexein^{R}X-350 (Inolex).
- Elastin-Hydrolysate (CTFA-Bezeichnung: Hydrolyzed Animal Elastin).
   Beispiele für erfindungsgemäß verwendbare Elastinhydrolysate sind die unter den Bezeichnungen Nutrilan^{R}Elastin E 20 (Henkel) und Gelita^{R} Gelastin (Deutsche Gelatine Fabriken Stoess & Co) vertriebenen Produkte. Hydrolysate aus Rinder-Elastin können bevorzugt sein.
- Keratin-Hydrolysate (CTFA-Bezeichnung: Hydrolyzed Animal Keratin).
   Beispiele für Markenprodukte sind Nutrilan^{R}Keratin W (Henkel), Kerapro^{R}S (Diedrichs), Promois^{R}WK H (Seiwa Oil & Chemetics) und Kerasol^{R} (Croda).
- Kollagenhydrolysat-Kondensate.
   Kollagenhydrolysate können erfindungsgemäß auch in Form ihrer Kondensate mit organischen Säuren, insbesondere Fettsäuren oder Fettsäuregemischen, eingesetzt werden. Bevorzugte Säuren sind Ölsäure, Myristinsäure, Undecylensäure, Kokosfettsäure und Abietinsäure. Die Kondensationsprodukte können auch in Form von Salzen, insbesondere Natrium-, Kalium- und Triethanolaminsalzen vorliegen. Solche Kondensationsprodukte tragen die CTFA-Bezeichnungen Oleoyl Hydrolyzed Animal Protein, Myristoyl Hydrolyzed Animal Protein, Oleoyl Hydrolyzed Animal Collagen, Potassium Coco Hydrolyzed Animal Protein, TEA Abietoyl Hydrolyzed Animal Collagen, Potassium Undecylenoyl Hydrolyzed Animal Collagen und TEA Coco Hydrolyzed Animal Collagen. Handelsprodukte sind beispielsweise Lamepon^{R}LPO, Lamepon^{R}4 SK, Lamepon^{R}UD, Lamepon^{R}460, Lamepon^{R}PA TR, Lamepon^{R}ST 40 und Lamepon^{R}S (Grünau) sowie Lexein^{R}A 240, Lexein^{R}S 620 und Lexein^{R}A 520 (Inolex).
- Elastinhydrolysat-Kondensate.
   Kondensationsprodukte von Elastinhydrolysaten mit Fettsäuren wie beispielsweise Laurinsäure (CTFA-Bezeichnung: Lauroyl Hydrolyzed Animal Elastin) können ebenfalls eingesetzt werden. Crolastin^{R}AS (Croda) ist ein entsprechendes Marktprodukt.
- Kationische Kollagenhydrolysate.
   Ein Beispiel für diese Verbindungsklasse ist das unter der Bezeichnung Lamequat^{R}L auf dem Markt befindliche Produkt (CTFA-Bezeichnung: Lauryldimonium Hydroxypropylamino Hydrolyzed Animal Protein; Grünau).

Weitere Proteine, deren Hydrolysate erfindungsgemäß verwendet werden können, sind Weizenproteine, Milchproteine, Eiweißproteine, Seidenproteine, Mandelproteine, Sojaeiweiß sowie Proteine aus Tierhäuten. Entsprechende Verkaufsprodukte sind beispielsweise Gluadin^{R}AGP (Henkel), Produkte der DiaMin^{R}-Serie (Diamalt), Lexein^{R}X-250 und Lexein^{R}MP (Inolex), Crotein^{R}A, Crotein^{R}C, Crotein^{R}ASC und Crotein^{R}O (Croda), Gelita^{R}flex S 50 (Deutsche Gelatine Fabrik Stoess & Co) und Hydrosilk^{R}10000 (Croda).

Auch diese Proteinhydrolysate können mit organischen Säuren oder Säuregemischen wie Myristinsäure, Ölsäure, Kokosfettsäure oder Abietinsäure zu erfindungsgemäß einsetzbaren Kondensaten umgesetzt werden. Beispiele für entsprechende Handelsprodukte sind Lexein^{R}A 200 (Inolex), Lamepon^{R}PO-TR, Lamepon^{R}PA-K, Lamepon^{R}S-MV und Lamepon^{R}S-TR (Grünau) und Crotein^{R}CCT (Croda).

Quaternisierte Produkte solcher Proteinhydrolysate sind beispielsweise als Lexein^{R}Q X 3000 (Inolex), Crotein^{R}Q (Croda) und Croquat ^{R} WKP (Croda) erhältlich.

Die Protein-Derivate sind in den erfindinngsgemäßen Mitteln bevorzugt in Mengen von 0,1 bis 10 Gew.-%, bezogen auf die Gesamtmenge des Mittels enthalten. Mengen von 0,1 bis 5 Gew.-% sind bevorzugt.

Weiterhin können die erfindungsgemäßen Mittel oberflächenaktive Verbindungen, insbesondere solche aus der Gruppe der anionischen, amphoteren, zwitterionischen und nichtionischen Tenside enthalten.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈-C₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkyiaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Vorzugsweise enthalten die erfindungsgemäßen Mittel die oberflächenaktiven Verbindungen in Mengen von 0,5 bis 10 Gew.-%, bezogen auf das jeweilige Mittel.

Weitere übliche Bestandteile der erfindungsgemäßen Wellmittel sind:
- Verdickungsmittel wie Agar-Agar, Guar-Gum, Alginate, Xanthan-Gum und Cellulose-Derivate wie Hydroxyethyl- und Methylhydroxypropyl-Cellulose,
- Strukturanten wie Glucose und Maleinsäure,
- Kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- Anionische, zwitterionische, amphotere und nichtionische Polymere wie beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyl-trimethylammoniumchlorid/Acrylat-Copolymere, Octylacrylamid/Methyl-methacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/ Vinylcaprolactam-Terpolymere sowie gegebenfalls derivatisierte Celluloseether.
- Lösungsvermittler, wie Ethanol, Isopropanol, Ethylenglykol, Propylenglykol, Glycerin und Diethylenglykol,
- Antischuppenwirkstoffe wie Piroctone Olamine und Zink Omadine,
- Substanzen zur Einstellung des pH-Wertes wie Natonlauge, Ammoniak, Ammoniumcarbonat und Citronensäure/Natriumcitrat-Puffer,
- Wirkstoffe wie Panthenol, Allantoin, Pyrrolidoncarbonsäuren, Pflanzenextrakte und weitere Vitamine,
- Lichtschutzmittel,
- Konsistenzgeber wie Zuckerester, Polyolester oder Polyolalkylether,
- Fette und Wachse, wie Walrat, Bienenwachs, Montanwachs, Paraffine und Fettalkohole,
- Überfettungsmittel wie polyethoxylierte Lanolinderivate, Lecithinderivate und Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Farbstoffe,
- Parfümöle, Dimethylisosorbid und Cyclodextrine,
- Perlglanzmittel wie Ethylenglykolmono- und -distearat,
- Treibmittel wie Propan-Butan-Gemische, N₂O, Dimethylether und Luft

Zwingender Bestandteil der Fixiermittel sind Oxidationsmittel, z. B. Natriumbromat, Kaliumbromat, Wasserstoffperoxid, und die zur Stabilisierung wäßriger Wasserstoffperoxidzubereitungen üblichen Stabilisatoren. Der pH-Wert solcher wäßriger N₂O₂-Zubereitungen, die üblicherweise etwa 0,5 bis 3,0 Gew.-% H₂O₂ enthalten, liegt bevorzugt bei 2 bis 4; er wird durch anorganische Säuren, bevorzugt Phosphorsäure, eingestellt. Fixiermittel auf Bromat-Basis enthalten die Bromate üblicherweise in Konzentrationen von 1 bis 10 Gew.-% eingesetzt und der pH-Wert der Lösungen wird auf 4 bis 7 eingestellt.

Bevorzugt werden die erfindungsgemäßen Fixiermittel als Feststoffe formuliert. Sie enthalten das Oxidationsmittel dann in Form eines Festkörpers, z.B. Kalium- oder Natriumbromat. Erst kurz vor der Anwendung werden diese Mittel dann mit Wasser versetzt. Ebenfalls möglich und bevorzugt ist, das Oxidationsmittel als 2-Komponenten-System zu formulieren. Die beiden Komponenten, von denen die eine bevorzugt eine Wasserstoffperoxidlösung oder eine wäßrige Lösung eines anderen Oxidationsmittels ist und die andere die übrigen Bestandteile enthält, werden ebenfalls erst kurz vor der Anwendung vermischt.

Neben den im Zusammenhang mit dem Wellmittel genannten weiteren Komponenten können die Fixiermittel als oberflächenaktive Verbindungen kationische Tenside enthalten.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide, insbesondere Chloride und Bromide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Alkylamidoamine, insbesondere Fettsäureamidoamine wie das unter der Bezeichnung Tego Amid^{R}S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind die sogenannten Esterquats wie die unter dem Warenzeichen Stepantex^{R} vertriebenen Dialkylammoniummethosulfate und Methyl-hydroxyalkyl-dialkoyloxyalkyl-ammoniummethosulfate.

Die erfindungsgemäßen Zwischenspülungen enthalten neben Wasser und den Tocopherolen im wesentlichen oberflächenaktive Verbindungen.

Sowohl Wellmittel als auch Fixiermittel können als Creme, Gel oder Flüssigkeit formuliert sein. Weiterhin ist es möglich, die Mittel in Form von Schaumaerosolen zu konfektionieren, die mit einem verflüssigten Gas wie z. B. Propan-Butan-Gemischen, Stickstoff, CO₂, Luft, N₂O, Dimethylether, Fluorchlorkohlenwasserstofftreibmitteln oder Gemischen davon in Aerosolbehältern mit Schaumventil abgefüllt werden.

### Beispiele

### I. Bestimmung der Reißfestigkeit

Zur Bestimmung der Reißfestigkeit wurden Haare der Firma Alkinco (#6221, "European dark grey") verwendet. Die einzelnen Haarsträhnen waren ca. 300 mg schwer und ca. 12 cm lang. Sie wurden doppelseitig abgebunden und der im folgenden beschriebenen Kaltwell-Behandlung unterzogen: Die Haare wurden bei 30°C 30 Minuten lang mit 50 ml Wellmittel behandelt, mit Wasser (38°C) gespült, 10 Minuten lang bei 30°C mit 50 ml einer 2 %igen Wasserstoffperoxidlösung fixiert und erneut mit Wasser (38°C) gespült. Danach wurde sofort an den noch nassen Haaren die Naßreißfestigkeit bestimmt. Zur Bestimmung der Trockenreißfestigkeit wurden die Haare nach der letzten Spülung 1 Stunde lang bei 40°C getrocknet.

Die Reißfestigkeit der nassen bzw. trockenen Haare wurde mit einer Zugdehnungs-Apparatur (Zwick, Typ 1454) bestimmt.

Die spezifische Reißarbeit wurde definiert als Quotient aus der Arbeit, die zum vollständigen Reißen der Haarsträhne aufgebracht werden mußte, und der Masse der Haarsträhne.

Die Wellmittel wurden direkt vor der Anwendung aus zwei Komponenten zusammengemischt:
Komponente 1: 31,5 g Thioglykolsäure wurden mit Ammoniak auf pH=9 eingestellt und mit Wasser auf 300 g aufgefüllt.
Komponente 2: 2,87 g Covitol^{R}F 1300¹ und 5,0 g Eumulgin^{R}B 1² wurden mit Wasser auf 50 g aufgefüllt.

¹ (pflanzliches D-α-Tocopherol (ca. 87 % Aktivsubstanz) (HENKEL CORP.))
² (Cetylstearylalkohol mit ca. 12 Mol Ethylenoxid (CTFA-Bezeichnung: Ceteareth-12) (HENKEL))

Sodann wurden die Komponenten 1 und 2 im Massenverhältnis 2:1 gemischt.

Der Vergleichsmischung fehlte die Komponente Covitol^{R}F 1300.

Die Wellmittel hatten somit folgende Zusammensetzungen (in Gew.-%):

| Komponenten | Erfindung (E) | Vergleich (V) |
|---|---|---|
| Thioglykolsäure | 7,0 | 7,0 |
| Eumulgin^{R}B | 3,3 | 3,3 |
| Covitol^{R}F 1300 | 1,7 | - |
| Ammoniak | ad pH=9.0 | ad pH=9.0 |
| Wasser | ad 100 | ad 100 |

Es wurden folgende Reißarbeiten bestimmt:

| nasses Haar | spezifische Reißarbeit [Ncm/g] | |
|---|---|---|
| | Wellmittel E | Wellmittel V |
| - 1 x kaltgewellt^{a} | 262,4 | 189,9 |
| - 3 x kaltgewellt^{b}^{,}^{d} | 168,8 | 123,9 |

| trockenes Haar | | |
|---|---|---|
| - 1 x kaltgewellt^{c} | 379,1 | 355,2 |

| | | |
|---|---|---|
| ^{a} Mittelwert aus 2 Meßreihen mit jeweils 11 Bestimmungen | | |
| ^{b} Mittelwert aus 22 Bestimmungen | | |
| ^{c} Mittelwert aus 11 Bestimmungen | | |
| ^{d} Die oben beschriebene Kaltwell-Behandlung wurde vor der Bestimmung insgesamt dreimal durchgeführt. | | |

Die Meßwerte zeigen eindeutig, daß die Reißfestigkeit der Haare signifikant größer war, wenn die Kaltwell-Behandlung mit einem erfindungsgemäßen Mittel durchgeführt wurde.

### II. Anwendungsbeispiele

Die Angaben in den folgenden Formulierungsbeispielen sind jeweils in Gewichtsprozent.

### 1) Wellmittel:

| | |
|---|---|
| Thioglykolsäure | 6,0 % |
| Ammoniumcarbonat | 3,0 % |
| Texapon^{R}K 1296³ | 3,0 % |
| Copherol^{R}1250⁴ | 2,0 % |
| Nutrilan^{R} Keratin W⁵ | 2,0 % |
| Ammoniak | ad pH=8 |
| Wasser | ad 100,0 % |

| | |
|---|---|
| ³ Natriumlaurylsulfat (96 % Aktivsubstanz, CTFA-Bezeichnung: Sodium Lauryl Sulfat) (HENKEL) | |
| ⁴ D-α-Tocopheryl-acetat (ca. 92 % Aktivsubstanz; CTFA-Bezeichnung: Tocopheryl Acetate) (HENKEL CORP.) | |
| ⁵ Hydrolysat aus Merinoschurwolle (CTFA-Bezeichnung: Hydrolyzed Keratin) (HENKEL) | |

### 2) Wellmittel:

| | |
|---|---|
| Thiomilchsäure | 6,0 % |
| Ammoniumcarbonat | 3,0 % |
| Texapon^{R}SB 3⁶ | 7,5 % |
| Copherol^{R}F 1300 | 2,0 % |
| Nutrilan^{R} I⁷ | 2,0 % |
| Ammoniak | ad pH=7,5 |
| Wasser | ad 100,0 % |

| | |
|---|---|
| ⁶ Dinatriumsalz eines Sulfobernsteinsäurehalbesters auf Basis eines Alkylpolyglykolethers (ca. 40 % Aktivsubstanz; CTFA-Bezeichnung: Disodium Laurethsulfosuccinate) (HENKEL) | |
| ⁷ Kollagenhydrolysat (ca. 39 % Aktivsubstanz; CTFA-Bezeichnung: Hydrolyzed Collagen) (HENKEL) | |

### 3) 2-Komponenten-Wellmittel

| Komponente 1: | |
|---|---|
| Dehyton^{R}G⁸ | 10,0 % |
| Ammoniumcarbonat | 1,0 % |
| Copherol^{R}F 1300 | 2,0 % |
| Harnstoff | 5,5 % |
| Ammoniak | ad pH=9,3 |
| Wasser | ad 100,0 % |

| | |
|---|---|
| ⁸ Fettsäureamidderivat mit amphoterem Charakter (Na-Salz des N-Hydroxyethyl-N-Kokosalkylamidoethyl-Glycinats (ca. 30 % Aktivsubstanz; CTFA-Bezeichnung: Cocoamphodiacetat)) (HENKEL) | |

| Komponente 2: | |
|---|---|
| Glycerinmonothioglykolat | 50,0 % |
| Wasser | 50,0 % |

Beim Zusammengeben beider Komponenten im Massenverhältnis von 4,5:1 ergibt sich ein Wellmittel mit einem pH-Wert von 7,2.

### 4) Wellmittel

| | |
|---|---|
| Natriumbisulfit | 6,0 % |
| Ammoniumcarbonat | 3,0 % |
| Texapon^{R}N 25⁹ | 10,0 % |
| Copherol^{R}F 1300 | 2,0 % |
| Gluadin^{R}Almond¹⁰ | 2,0 % |
| Ammoniak | ad pH=8 |
| Wasser | ad 100,0 % |

| | |
|---|---|
| ⁹ Natriumlaurylethersulfat (ca. 28 % Aktivsubstanz; CTFA-Bezeichnung: Sodium Laureth Sulfate) (HENKEL) | |
| ¹⁰ Proteinhydrolysat aus Mandelmehl (22 % Aktivsubstanz; CTFA-Bezeichnung: Hydrolyzed Almond Protein) (HENKEL) | |

### 5) Wellmittel

| | |
|---|---|
| Thioglykolsäure | 6,0 % |
| Ammoniumcarbonat | 3,0 % |
| Texapon^{R}K 1296 | 3,0 % |
| Copherol^{R}1250 | 2,0 % |
| Gafquat^{R}755¹¹ | 2,5 % |
| Parfümöl | q.s. |
| Ammoniak | ad pH=8 |
| Wasser | ad 100,0 % |

| | |
|---|---|
| ¹¹ quaternisiertes Poly(vinylpyrrolidon-dimethylaminoethylmethacrylat) (19 % Aktivsubstanz in Wasser) (GAF) | |

### 6) Wellmittel

| | |
|---|---|
| Thioglykolsäure | 6,0 % |
| Ammoniumcarbonat | 3,0 % |
| Texapon^{R}K 1296 | 3,0 % |
| Copherol^{R}1250 | 2,0 % |
| Acrylamidopropyl-trimethylammoniumchlorid-Acrylsäure-Copolymer | 0,5 % |
| Parfümöl | q.s. |
| Ammoniak | ad pH=8 |
| Wasser | ad 100,0 % |

### 7) Wellmittel

| | |
|---|---|
| Thioglykolsäure | 6,0 % |
| Alkylglykosid¹² | 2,0 % |
| Ammoniumcarbonat | 3,0 % |
| Texapon^{R}K 1296 | 3,0 % |
| Copherol^{R}1250 | 2,0 % |
| Parfümöl | q.s. |
| Ammoniak | ad pH=8 |
| Wasser | ad 100,0 % |

| | |
|---|---|
| ¹² Alkylglykosid auf Basis Glucose mit C_{12/14}-Alkylresten und einem mittleren Oligomerisierungsgrad von 1,4 | |

### 8) Zwischenspülung

| | |
|---|---|
| Cremophor^{R}RH 40¹³ | 0,4 % |
| Kelzan^{R} ¹⁴ | 0,5 % |
| Copherol^{R}1250 | 2,0 % |
| Wasser | ad 100,0 % |

| | |
|---|---|
| ¹³ hydriertes Rizinusöl mit 45 Mol Ethylenoxid pro Mol Rizinusöl umgesetzt (CTFA-Bezeichnung: PEG-40-Hydrogenated-Castor-Oil) (BASF) | |
| ¹⁴ Xanthan-Gum (KELCO) | |

### 9) Fixiermittel

| | |
|---|---|
| Wasserstoffperoxid (50 %) | 4,5 % |
| Eumulgin^{R}B 1 | 4,0 % |
| Copherol^{R}1250 | 1,5 % |
| Nutrilan^{R} I | 2,0 % |
| Luviquat^{R}FC-550¹⁵ | 2,0 % |
| Parfümöl | q.s. |
| Wasser | ad 100,0 % |

| | |
|---|---|
| ¹⁵ Vinylimidazoliniummethochlorid-Vinylpyrrolidon-Copolymerisat (50:50) (40 % Aktivsubstanz) (BASF) | |

## Patentansprüche

1. Verfahren zur dauerhaften Verformung von Keratinfasern, bei welchem man die Faser vor und/oder nach einer mechanischen Verformung mit einer wäßrigen Zubereitung einer keratinreduzierenden Substanz behandelt, nach einer Einwirkungszeit mit einer ersten Spülung spült, dann mit einer wäßrigen Zubereitung eines Oxidationsmittels fixiert und ebenfalls nach einer Einwirkungszeit spült, dadurch gekennzeichnet, daß in mindestens einer der beiden wäßrigen Zubereitungen oder in der ersten Spülung ein Tocopherol oder ein Ester eines Tocopherols mit einer organischen oder anorganischen Säure enthalten ist.

2. Verfahren nach Anspruch 1, dadurch gekennzeichnet, daß das Tocopherol oder der Tocopherol-Ester ausgewählt ist aus α-Tocopherol, α-Tocopherolacetat, α-Tocopherolpalmitat und α-Tocopherolsuccinat.

3. Verfahren nach einem der Ansprüche 1 oder 2, dadurch gekennzeichnet, daß das Tocopherol oder der Tocopherol-Ester in einer Menge von 0,1 bis 10, insbesondere von 1 bis 3 Gew.-%, bezogen auf die jeweilige Zubereitung, enthalten ist.

4. Verfahren nach einem der Ansprüche 1 bis 3, dadurch gekennzeichnet, daß das Tocopherol und/oder der Tocopherol-Ester in der wäßrigen Zubereitung einer keratinreduzierenden Substanz enthalten ist.

5. Verfahren nach einem der Ansprüche 1 bis 4, dadurch gekennzeichnet, daß das Tocopherol und/oder der Tocopherol-Ester sowohl in der wäßrigen Zubereitung einer keratinreduzierenden Substanz als auch in der ersten Spülung oder der wäßrigen Zubereitung eines Oxidationsmittels enthalten ist.

6. Mittel zur Durchführung der reduzierenden Stufe eines Verfahrens zur dauerhaften Verformung von Keratinfasern enthaltend übliche Bestandteile, dadurch gekennzeichnet, daß es zusätzlich ein Tocopherol oder einen Ester eines Tocopherols mit einer organischen oder anorganischen Säure enthält.

7. Mittel zur Durchführung der oxidierenden Stufe eines Verfahrens zur dauerhaften Verformung von Keratinfasern enthaltend übliche Bestandteile, dadurch gekennzeichnet, daß es zusätzlich ein Tocopherol oder einen Ester eines Tocopherols mit einer organischen oder anorganischen Säure enthält.

8. Mittel zum Spülen nach Durchführung der reduzierenden Stufe eines Verfahrens zur dauerhaften Verformung von Keratinfasern enthaltend übliche Bestandteile, dadurch gekennzeichnet, daß es zusätzlich ein Tocopherol oder einen Ester eines Tocopherols mit einer organischen oder anorganischen Säure enthält.

9. Mittel nach einem der Ansprüche 6 bis 8, dadurch gekennzeichnet, daß das Tocopherol oder der Tocopherol-Ester ausgewählt ist aus α-Tocopherol, α-Tocopherolacetat, α-Tocopherolpalmitat und α-Tocopherolsuccinat.

10. Mittel nach einem der Ansprüche 6 bis 9, dadurch gekennzeichnet, daß das Tocopherol oder der Tocopherol-Ester in einer Menge von 0,1 bis 10, insbesondere von 1 bis 3 Gew.-%, bezogen auf das gesamte Mittel, enthalten ist.

11. Mittel nach einem der Ansprüche 6 bis 10, dadurch gekennzeichnet, daß es zusätzlich ein Proteinderivat, ausgewählt aus Proteinhydrolysaten, quaternierten Proteinhydrolysaten und Proteinhydrolysat-Fettsäure-Kondensationsprodukten enthält.

12. Mittel nach einem der Ansprüche 6 bis 11, dadurch gekennzeichnet, daß es eine oberflächenaktive Verbindung enthält.

## Claims

1. A process for permanently deforming keratin fibres, in which the fibres are treated with an aqueous preparation of a keratin-reducing substance before and/or after being mechanically deformed, are rinsed with a first rinse after a contact time, are then fixed with an aqueous preparation of an oxidizing agent and are again rinsed after a contact time, characterized in that a tocopherol or an ester of a tocopherol with an organic or inorganic acid is present in at least one of the two aqueous preparations or in the first rinse.

2. A process as claimed in claim 1, characterized in that the tocopherol or the tocopherol ester is selected from α-tocopherol, α-tocopherol acetate, α-tocopherol palmitate and α-tocopherol succinate.

3. A process as claimed in claim 1 or 2, characterized in that the tocopherol or the tocopherol ester is present in a quantity of 0.1 to 10% by weight and more particularly in a quantity of 1 to 3% by weight, based on the particular preparation.

4. A process as claimed in any of claims 1 to 3, characterized in that the tocopherol and/or the tocopherol ester is present in the aqueous preparation of a keratin-reducing substance.

5. A process as claimed in any of claims 1 to 4, characterized in that the tocopherol and/or the tocopherol ester is present both in the aqueous preparation of a keratin-reducing substance and in the first rinse or the aqueous preparation of an oxidizing agent.

6. A formulation for carrying out the reducing step of a process for permanently deforming keratin fibres containing typical constituents, characterized in that it additionally contains a tocopherol or an ester of a tocopherol with an organic or inorganic acid.

7. A formulation for carrying out the oxidizing step of a process for permanently deforming keratin fibres containing typical constituents, characterized in that it additionally contains a tocopherol or an ester of a tocopherol with an organic or inorganic acid.

8. A formulation for rinsing after carrying out the reducing step of a process for permanently deforming keratin fibres containing typical constituents, characterized in that it additionally contains a tocopherol or an ester of a tocopherol with an organic or inorganic acid.

9. A formulation as claimed in any of claims 6 to 8, characterized in that the tocopherol or the tocopherol ester is selected from α-tocopherol, α-tocopherol acetate, α-tocopherol palmitate and α-tocopherol succinate.

10. A formulation as claimed in any of claims 6 to 9, characterized in that the tocopherol or the tocopherol ester is present in a quantity of 0.1 to 10% by weight and more particularly in a quantity of 1 to 3% by weight, based on the formulation as a whole.

11. A formulation as claimed in any of claims 6 to 10, characterized in that it additionally contains a protein derivative selected from protein hydrolyzates, quaternized protein hydrolyzates and protein hydrolyzate/fatty acid condensates.

12. A formulation as claimed in any of claims 6 to 11, characterized in that it contains a surface-active compound.

## Revendications

1. Procédé de déformation permanente des fibres kératiniques, dans lequel on traite les fibres avant et/ou après une déformation mécanique par une préparation aqueuse d'une substance réduisant la kératine, on les rince par un premier rinçage après une durée d'action, on les fixe à l'aide d'une préparation aqueuse d'un oxydant et on les rince également après une durée d'action, caractérisé en ce qu'un tocophérol ou un ester d'un tocophérol avec un acide organique ou inorganique est contenu dans au moins une des deux préparations aqueuses ou dans le premier rinçage.

2. Procédé selon la revendication 1, caractérisé en ce que le tocophérol ou l'ester de tocophérol est sélectionné parmi l'α-tocophérol, l'acétate d'α-tocophérol, le palmitate d'α-tocophérol et le succinate d'α-tocophérol.

3. Procédé selon la revendication 1 ou 2, caractérisé en ce que le tocophérol ou l'ester de tocophérol est contenu dans une proportion de 0,1 à 10, en particulier de 1 à 3 % en poids, par rapport à la préparation correspondante.

4. Procédé selon une des revendications 1 à 3, caractérisé en ce que le tocophérol et/ou l'ester de tocophérol est contenu dans la préparation aqueuse d'une substance réduisant la kératine.

5. Procédé selon une des revendications 1 à 4 caractérisé en ce que le tocophérol et/ou l'ester de tocophérol est contenu aussi bien dans la préparation aqueuse d'une substance réduisant la kératine que dans le premier rinçage ou dans la préparation aqueuse d'un oxydant.

6. Agent pour la réalisation de l'étape de réduction d'un procédé de déformation permanente des fibres de kératine, renfermant les constituants usuels, caractérisé en ce qu'il contient en plus un tocophérol ou un ester d'un tocophérol avec un acide organique ou inorganique.

7. Agent pour la réalisation de l'étape d'oxydation d'un procédé de déformation permanente des fibres de kératine, renfermant les constituants usuels, caractérisé en ce qu'il contient en plus un tocophérol ou un ester d'un tocophérol avec un acide organique ou inorganique.

8. Agent pour le rinçage après la réalisation de l'étape de réduction d'un procédé de déformation permanente des fibres de kératine, renfermant les constituants usuels, caractérisé en ce qu'il contient en plus un tocophérol ou un ester d'un tocophérol avec un acide organique ou inorganique.

9. Agent selon une des revendications 6 à 8, caractérisé en ce que le tocophérol ou l'ester de tocophérol est sélectionné parmi l'α-tocophérol, l'acétate d'α-tocophérol, le palmitate d'α-tocophérol et le succinate d'α-tocophérol.

10. Agent selon une des revendications 6 à 9, caractérisé en ce que le tocophérol ou l'ester de tocophérol est contenu dans une proportion de 0,1 à 10, en particulier de 1 à 3 % en poids, par rapport à la totalité de l'agent.

11. Agent selon une des revendications 6 à 10, caractérisé en ce qu'il contient en plus un dérivé protéinique, sélectionné parmi les hydrolysats de protéines, les hydrolysats de protéines quaternisés et les produits de condensation d'hydrolysats de protéines avec des acides gras.

12. Agent selon une des revendications 6 à 11, caractérisé en ce qu'il contient un composé tensioactif.
